# EUROPEAN PATENT SPECIFICATION

(11) **EP 3 316 714 B1**
(45) Date of publication and mention of the grant of the patent: **21.11.2018**
(21) Application number: 17719010.5
(22) Date of filing: 07.04.2017
(51) Int. Cl.: A24F 47/00, H05B 1/02

(54) **ELECTRONIC CIGARETTE**
ELEKTRONISCHE ZIGARETTE
CIGARETTE ÉLECTRONIQUE

(30) Priority: 13.04.2016 US 201662321807 P
(43) Date of publication of application: 09.05.2018
(60) Divisional application: 18195400.9
(73) Proprietor: MD&C Creative Maison SA, 1206 Geneva (CH)
(72) Inventor: WENDT THEVENAZ, Cristina, CH-1206 Geneva (CH); DANIELE KILLY, Maria, CH-1206 Geneva (CH); FOURNIER, Tom, 69009 Lyon (FR)
(74) Representative: Serjeants LLP
(86) International application number: PCT/IL2017/050429
(87) International publication number: WO 2017/179043

(56) References cited:
- EP-A1- 2 965 642
- US-A- 5 666 977
- US-A1- 2015 209 530
- US-B2- 8 251 060

## Description

### TECHNOLOGICAL FIELD

The present application is directed to an electronic cigarette, in particular, an electronic cigarette comprising a heating element in the reusable part, and including additional user features.

### BACKGROUND

Electronic cigarettes have become quite common in recent years in replacing regular cigarettes, providing a healthier alternative to common tobacco-based substances without considerably affecting the smoking experience.

An electronic cigarette commonly comprises a base part accommodating all the operating components of the electronic cigarette (processor, battery etc.), and a disposable part which contains the smoked substance filling, and which is discarded/refilled after a given period of use.

In general, the base part comprises the core components of the electronic cigarette which are configured for vaporizing the smoked substance which resides, in a liquid state, in the disposable part. For this purpose, a heating element (e.g., a coil) is commonly implemented in the disposable part which is electrically wired to the battery once the disposable part and base part are assembled, and which is configured for transferring heat to the smoked substance for the purpose of its vaporization.

Exemplary patents dealing with heating element in the cartridge are based on:
- heating element in atomizing component in cartridge pipe (Shenzhen smoore technology Ltd., Electronic cigarette, WO2015043126);
- heating element and e-liquid to constitute the atomizer (SIS Resources Ltd. WO2015028891); and
- two heating elements gathering in the same area (the aerozolisation zone) 2 vaporized liquids from 2 different reservoirs. Transport elements are entangled with heating element, and create the aerozolisation zone. (R.J. Reynolds Tobacco Co. US 2014/0000638).

Exemplary patents dealing with heating in the disposable part are based on impregnation of a mesh and/or roving with e-liquid and heat it by a separated heated coiled. Heated coil is placed in a disposable part (with the battery) and the cartridge is in the disposable part. Hence there is no vaporization chamber (distance between mesh and liquid storage, see Ruyan investment holdings Ltd., Electronic cigarette WO2013155645).

US 2015/0209530 discloses a vaporizer device configured to heat a substrate located within a substrate holder. The vaporizer in US 2015/0209530 comprises a heat generator and a heat conductor.

### GENERAL DESCRIPTION

In accordance with one aspect of the subject matter of the present application, there is provided an electronic cigarette as defined in claim 1, comprising a base part and a cartridge configured to be assembled thereto, said base part comprising: a battery and at least one heating element connected to the battery, and said cartridge comprising a substance chamber configured for containing therein a substance to be smoked, said substance chamber comprising a substance outlet; a vaporization chamber in communication with the substance outlet for receiving said substance therein; and at least one heat transfer unit configured, at least when the cartridge is assembled to the base part, for being heated by the heating element of the base part and for heating to the substance being provided via said substance outlet for its vaporization in the vaporization chamber.

In accordance with another aspect of the subject matter of the present application, there is provided a cartridge for an electronic cigarette, said cartridge being configured to be assembled with a base part of the electronic cigarette having a battery and at least one heating element connected to the battery, said cartridge comprising: a substance chamber configured for containing therein a substance to be smoked, said substance chamber comprising a substance outlet; a vaporization chamber in communication with the substance outlet for receiving said substance therein; and at least one heat transfer unit configured, at least when the cartridge is assembled to the base part, for being heated by the heating element of the base part and for heating the substance being provided via said substance outlet for its vaporization in the vaporization chamber.

The substance to be smoked will hereinafter be referred to as 'smoked substance'. It should be understood that the term 'smoked substance' does not necessitate actual smoking and merely refers to the substance (e.g., liquid) which, eventually, may be vaporized for providing the user with a smoking experience.

Under the above arrangement, the heating element does not come into direct contact with the substance chamber and, more importantly, with the smoked substance itself.

The cartridge can further comprise a sorption member at least partially disposed within the vaporization chamber. Under such a design, the heat is delivered from the heat transfer unit to the sorption member by conduction.

The sorption member can be made of any material or a combination of materials being able to perform sorption and/or absorption of another material, and can be made, for example, of one or more of the following materials: fiber, glass, aluminum, cotton, ceramic, cellulose, glass fiber wick, stainless steel mesh, polyethylene (PE), polypropylene, polyethylene terephthalate (PET), poly(cyclohexanedimethylene terephthalate) (PCT), polybutylene terephthalate (PBT), polytetrafluoroethylene (PTFE), expanded polytetrafluoroethylene (ePTFE), and BAREX®, etc.

The heat transfer unit can have a first side configured, when the cartridge is assembled to the base part, for interfacing the heating element for receiving heat therefrom, and a second side facing the substance outlet for providing heat to the substrate being provided from the substrate outlet.

When the cartridge is assembled to the base part, the heating element can be separated from the vaporization chamber by the heat transfer unit.

The cartridge further comprises a porous member interposed between the substance outlet and the vaporization chamber, and configured for allowing passage of said substance from said substance chamber to said vaporization chamber.

The porous member can be interposed between the substance outlet and the heat transfer unit. The porous member can be also interposed between the substance chamber and the vaporization chamber. The porous member and the sorption member can be integrated the same member. The sorption member can at least partially be surrounded by or disposed within the porous member. The porous member can provide protection to the sorption member and/or hold the sorption member. The porous member can be formed as a mesh. The porous member can be made, for example, from stainless steel, or any other similar material which is able to transfer substance.

The vaporization chamber can be interposed between the porous member and the heat transfer unit.

The porous member can have a first surface associated with the substance outlet for absorbing the substance, and a second surface associated with the heat transfer unit, for allowing passage of the substance from the substance chamber to the vaporization chamber. The first surface can be immersed in the substance.

The second surface can be associated with the heat transfer unit by facing the heat transfer unit and can be separated from the heat transfer unit by the vaporization chamber for allowing introduction of the substance from the second surface into the vaporization chamber for its heating and thereby its vaporization.

In accordance with one design embodiment, the arrangement can be such that the second surface of the porous member does not come into direct contact with the heat transfer unit, thereby minimizing any chance of damage to the porous member by the heat transfer unit.

The porous member can be formed with a hole extending therethrough between the first surface and the second surface and establishing fluid communication between the vaporization chamber and a mouthpiece of the cartridge via a vaporization channel, thereby allowing the vaporized substance to escape from the vaporization chamber via the mouthpiece. The hole can constitute at least part of the vaporization chamber.

In accordance with another design embodiment, the second surface of the porous member can be at least partially in direct contact with the heat transfer unit, thereby increasing the efficiency of heat transfer to the sorption member, thereby increasing vaporization of the smoked substance. Under such a design, the heat is delivered from the heat transfer unit to the second surface of the porous member by conduction.

The sorption member can have an elongated form (e.g. a string, strip etc.).

The sorption member can be in the form of a flat cap, cover or cushion of soft material. In particular, the sorption member can be sized and shaped to fit the cross-section of one of the vaporization chamber and substance chamber. The porous member can be sized and shaped to fit the cross-section of one of the vaporization chamber and substance chamber.

The cartridge can further comprises: a cartridge housing at least partially including said substance chamber and said vaporization chamber, and a vaporization channel extending along the cartridge housing and in fluid communication with the vaporization chamber. The cartridge housing can have a proximal end configured with a mouthpiece which is in fluid communication with the vaporization chamber via the vaporization channel and a distal end associated with the heat transfer unit. The mouthpiece is configured for providing the vaporized substance to the user. The heat transfer unit can be disposed at the distal end. The heat transfer unit can be perpendicular to the vaporization channel.

The substance chamber can be juxtaposed with the vaporization channel extending between the vaporization chamber and the mouthpiece. The substance chamber can be disposed at the surrounding of the vaporization channel.

The cartridge housing can comprise a first air inlet formed therein for establishing fluid communication between an exterior of the cartridge and the vaporization chamber for allowing passage of air into the vaporization chamber for its vaporization therein with the substance.

The first air inlet can be disposed in proximity to the distal end of the cartridge.

The base part can have a base housing having an interfacing portion associated with the heating element and configured for interfacing the distal end of the cartridge housing when the cartridge is assembled to the base part, so as to bring the heating element into proximity or contact with the heat transfer unit.

The interfacing portion can comprise a second air inlet formed therein, and configured at least when the cartridge is assembled to the base part for establishing fluid communication between an exterior of the electronic cigarette and the vaporization chamber via the first air inlet for allowing passage of air into the vaporization chamber for its vaporization therein with the substance. When the cartridge is assembled to the base part, the first air inlet and the second air inlet can be inline with each other.

In assembly, the cartridge is attached to the base part so that the heat transfer unit comes into contact with the heating element to receive heat therefrom by conduction.

In operation, upon turning on the electrical cigarette, the battery electrically heats the heating element of the base part, which then provides its heat by conduction to the heating element. This, in turn, provides the heat the sorption member, entailing vaporization of the substance absorbed therein.

As this process is continuous, the substance from the substance chamber keeps filling (owing to capillarity absorbing effects) the sorption member, thereby providing a constant supply of substance thereto. Substance vapors created during the above process are forced to escape the cartridge via the vaporization channel leading to the mouthpiece and to the user.

The heat transfer unit can be made of thermally conductive material, for example, metals (e.g., aluminum, copper, etc.).

The cartridge housing can be made of one or more of the following materials: aluminium, polyether ether ketone (PEEK), polyimides, such as Kapton®, polyethylene terephthalate (PET), polyethylene (PE), high-density polyethylene (HDPE), polypropylene (PP), polystyrene (PS), fluorinated ethylene propylene (FEP), polytetrafluoroethylene (PTFE), polyoxymethylene (POM), polybutylene terephthalate (PBT), Acrylonitrile butadiene styrene (ABS), Polycarbonates (PC), epoxy resins, polyurethane resins and vinyl resins. The parts of the cartridge housing can assembled or at least partially overmoulded.

The heating element can be made of an electrically resistive material. The heating element can comprise a non-elastic material, for example a ceramic sintered material, such as alumina (AI2O3) and silicon nitride (S13N4), or printed circuit board or silicon rubber. The heating element can comprise an elastic, metallic material, for example an iron alloy or a nickel-chromium alloy.

The electrically resistive material of the heating element can include one or more of the following materials: semiconductors such as doped ceramics, electrically "conductive" ceramics (such as, for example, molybdenum disilicide), carbon, graphite, metals, metal alloys and composite materials made of a ceramic material and a metallic material. Such composite materials may comprise doped or undoped ceramics. Examples of suitable doped ceramics include doped silicon carbides. Examples of suitable metals include titanium, zirconium, tantalum and metals from the platinum group. Examples of suitable metal alloys include stainless steel, nickel-, cobalt-, chromium-, aluminum-titanium-zirconium-, hafnium-, niobium-, molybdenum-, tantalum-, tungsten-, tin-, gallium-and manganese-alloys, and super-alloys based on nickel, iron, cobalt, stainlesssteel, Timetal® and iron-manganese-aluminum based alloys. Timetal® is a registered trademark of Titanium Metals Corporation, 1999 Broadway Suite 4300, Denver, Colorado. In composite materials, the electrically resistive material may optionally be embedded in, encapsulated or coated with an insulating material or vice-versa, depending on the kinetics of energy transfer and the external physicochemical properties required.

The heating element may be formed using a metal having a defined relationship between temperature and resistivity. In such embodiments, the metal may be formed as a track between two layers of suitable insulating materials. An heating element formed in this manner may be used both as a heater and a temperature sensor.

The heating element can include a temperature sensor embedded therein or attached thereto.

The heating element can be at least partially encapsulated in or coated with a protecting material, such as glass.

The battery can be a DC voltage source. For example, the battery can be a Nickel-metal hydride battery, a Nickel cadmium battery, or a Lithium based battery, for example a Lithium-Cobalt, a Lithium-Iron-Phosphate, a Lithium-Ion or a Lithium-Polymer battery.

Throughout the entire process, neither the substance nor the sorption member comes into any contact with the heating element, as they are separated at least by the heat transfer unit. In other words, the second surface of the sorption member heats up owing only to its association with the heat transfer unit.

Such a configuration has at least the following advantages:
- reducing the risk of corrosion process on the heating element, modification of vaporization temperature and decrease in heater efficiency;
- reducing the risk of liquid ingestion with unperfected vaporization process, or heating element with filter addition;
- reducing exposure to fouling, steams properties modifications and cartridge cost increase;
- reducing risk of explosion with unperfected vaporization based on pressure devices; and
- reducing flammability risk with the contact between sorption member and the heating element (as known in the art).

It should also be noted that since the heating element is accommodated within the base part, which is reusable, it allows using a higher grade heating element, with better safety characteristics and heating capacity and efficiency, something that is not economically viable when the heating element is accommodated within the disposable part (i.e., the cartridge).

In addition, the elimination of contact between the smoked substance and the heating element allows, for all practical purposes, considerably reducing the chance of any residual taste from being transferred between refills/replacements of the cartridge.

The base part can further comprise a processor associated with electrical components of the electronic cigarette, including the battery. The electrical components can be configured for providing additional features to the electronic cigarette, some of which are:
- a sensing unit (e.g., a gyroscope, an accelerometer, a compass, etc.) connected to the processor for identifying movement or position of the electronic cigarette and thereby allow a gesture-based operation thereof. In case of an internal gyroscope, it can be constituted by or supplemented by any other sensor like a compass or an accelerometer configured to detect gestures. For example, the electronic cigarette can be tipped twice to be 'switched-on' and/or waived to the side to indicate switching off; a puff detector based on a microphone or a pressure sensor, connected to processor. In an example in which the puff detector is a microphone, when a user breathes in, it produces a specific vibration used to count puffs. The puff detector can detect the power with which the user breathes and thereby automatically adapt the quantity of the delivered smoked substance;
- cigarette equivalency measure - allowing the processor to determine, based on the number of puffs and length of each puff, what is the estimated number of corresponding 'real life cigarettes' smoked by the user and alert him to the fact. For example, the processor can be configured to provide a signal to the user that he has smoked over ten cigarettes and set an alert. Alternatively, the user may be able to set, in advance, a cigarette limit after which the electronic cigarette shuts down for a predetermined amount of time;
- electronic turns off the cigarette, when the puff number or/and defined puffing time breathed in is equivalent to a defined puff number or/and defined puffing time.

In general, the subject matter of the present application provides at least the following advantages:
- simple flavor replacement by replacing one cartridge with another cartridge;
- contact prevention between the substance in the cartridge, and the heating element, avoiding flavor transfer from one cartridge to another; - prevention of contact between the emerged sorption member and the heating element, decreasing risk roving fire ignition; and
- allowing use of a reusable opened (or ongoing) cartridge.

In accordance with another aspect of the subject matter of the present application, there is provided an electronic cigarette comprising a base part and a cartridge configured to be assembled thereto, said base part comprising a battery, a processor associated with the battery and one or more orientation components associated with the processor, said orientation component being configured for identifying movement, orientation or spatial position of at least the base part and providing data regarding said movement to the processor, based on which said processor is configured for at least turning the battery on/off. It is appreciated that based on different gestures performed by the user, e.g. orienting the electronic cigarette in a certain manner or moving it in a specific direction, the processor can provide additional operational modes of the electronic cigarette, e.g. standby mode, regulation of vapor amount, timed smoking programs, etc. The electronic cigarette can be configured for being turned on by a predetermined shaking motion thereof by a user

In accordance with yet another aspect of the subject matter of the present application, there is provided an electronic cigarette comprising a base part configured to be assembled to a cartridge including a substance, said base part comprising a battery, and a processor associated with the battery, and at least one sensory component associated with the cartridge and with the processor, said sensory component being configured for obtaining at least one smoking parameter from the cartridge and providing said data to the processor, and wherein said processor is configured, at least during operation of the cigarette, for calculating a second smoking parameter based on said first smoking parameter and provide a corresponding output to either the user or the battery.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig. 1** is a schematic cross-sectional view of an electronic cigarette according to one example of the presently disclosed subject matter;
**Fig. 2A** is a schematic cross-section of one example of a cartridge used in the electronic cigarette shown in Fig. 1;
**Fig. 2B** is a schematic cross-section of another example of a cartridge used in the electronic cigarette shown in Fig. 1;
**Fig. 2C** is a schematic cross-section of yet another example of a cartridge used in the electronic cigarette shown in Fig. 1;
**Fig. 3A** is a schematic cross-sectional view of a disassembled electronic cigarette according to another example of the present disclosed subject matter; and
**Fig. 3B** is a schematic cross-sectional view of the electronic cigarette of Fig. 3A in its assembled form.

### DETAILED DESCRIPTION OF EMBODIMENTS

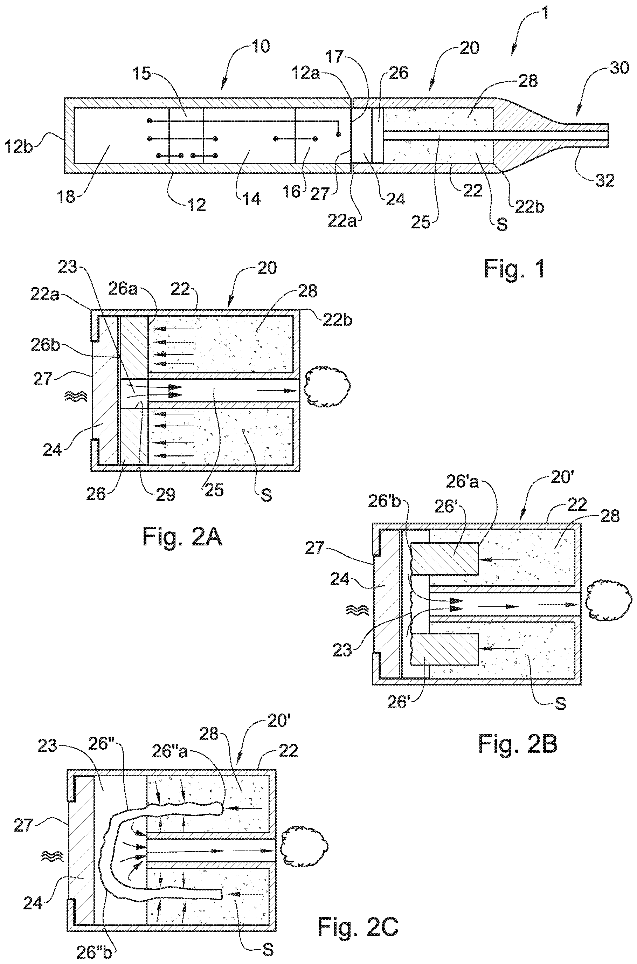

Attention is first drawn to Fig. 1 in which a schematic cross-section of the electronic cigarette of the present application is shown, generally designated **1** and comprising a base part **10** and a cartridge **20** attached to one another.

The base part **10** comprises a cylindrical housing **12** accommodating therein a battery **14** connected to a heating element **16** located at a first end **12a** of the housing **12** and having an interface surface **17** configured for matching a corresponding interface surface **27** of the cartridge. The battery **14** is configured for providing the heating element **16** with the necessary power for its operation, allowing it to become heated to a required temperature.

The battery **14** is also connected to a processor unit **18** and a sensor **15,** providing the required power for their operation. The processor unit **18** is connected to both the sensor **15** and the heating element **16**. Both processor unit **18** and the sensor **15** are located, in this specific example, on an opposite side of the battery **14** to that of the heating element **16**, wherein the battery **14** acts as a divider between the heating element **16** and other sensitive components of the electronic cigarette **1.** However, this arrangement is not compulsory and any other arrangement of the components within the base part **10** may be applicable.

In addition, the sensor **15** can be configured for identifying movement, orientation and spatial position of at least the base part **10**, and providing this information (hereinafter: spatial data) to the processor **18**. Based on the spatial data, the processor **18** can determine whether to turn the heating element **16** on/off, and/or if to set a certain operational regime of the electronic cigarette **1**, e.g. standby mode, regulation of vapor amount, timed smoking programs etc.

Thus, in operation, upon identification of a predetermined gesture of the user by the sensor **15**, this information is provided from the sensor **15** to the processor **18**, which then determines, based on said gesture, the proper operational mode of the electronic cigarette **1.** Upon determining such a mode, the processor **18**, which is directly linked to the heating element **16**, provides the latter with the proper operational signal determining the required temperature and heating rate of the heating element **16**, while the power for such heating is supplied by the battery **14.**

As a result, the electronic cigarette **1** can be configured for being operated using gestures and positions thereof, for example, a double-shake of the electronic cigarette **1** can indicate to the processor to turn the electronic cigarette **1** on/off where as holding the electronic cigarette **1** in a vertical orientation can indicate the processor to set a 'standby' mode for the electronic cigarette **1**. It should be appreciated that the sensor can be of various types (e.g. sound, motion, orientation, acceleration etc.), whereby the gestures required from the user in order to operate the electronic cigarette can change correspondingly, e.g. (knocking the electronic cigarette against a surface, speed of motion eng.).

With additional reference being made to Fig. 2A, the cartridge **20** of the electronic cigarette **1** comprises a cartridge housing **22** accommodating a heat transfer unit **24** located at a distal end **22a** of the cartridge housing **22** and having the interface surface **27** configured for matching a corresponding interface surface **17** of the heating element **16**, when the cartridge **20** is attached to the base **10.** At an opposite proximal end **22b** of the cartridge housing **22**, the cartridge **20** comprises a mouthpiece **30**, configured for being introduced into a user's mouth (not shown).

The housing **22** is formed with a substance chamber **28** configured for containing therein a flavored substance S to be smoked by the user. The chamber **28** is extending between the proximal end **22b** and the distal end **22a**, but is spaced from the distal end **22a.** The substance chamber **28** surrounds a vaporization chamber **23** formed as hole therein. The vaporization chamber **23** coextends with a vaporization channel **25** which coextends with a mouthpiece channel **32** of the mouthpiece **30.**

The housing **20** further accommodates therein a sorption member **26** juxtaposed between the heat transfer unit **24** and the substance chamber **28** The sorption member **26** is configured, on the one hand, for absorbing therein some of the substance S, and, on the other hand, for being heated by the heat transfer unit **24** thereby allowing the substance S absorbed therein to vaporize into the vaporization chamber **23** and escape from the vaporization chamber **23** via the vaporization channel **25** to the user via the mouthpiece **30**. Although not shown in Fig. 2A, the vaporization chamber **23** is in fluid communication with the exterior of the electronic cigarette **1** for receiving air thereto when the user inhales the smoked substance via the mouthpiece **30**.

In operation, the heating element **16** is heated up by the battery and provides it heat to the heat transfer unit **24** via conduction between the interface surfaces **17, 27**. Thus, the sorption member **26** is heated indirectly via the heat transfer unit **24** and not directly via the heating element, leading to considerable advantages in terms of safety, temperature control and reliability of the electronic cigarette **1.**

As a result of heating of the sorption member **26**, the substance S absorbed therein vaporizes in the vaporization chamber **23** and the vapor escapes the cartridge **20** via the vaporization channel **25** as indicated by the arrows.

It is important to note that in this example, the sorption member **26** takes up substantially all the space between the substance chamber **28** and the heat transfer unit **24** so that it has a first surface **26a** configured for absorbing the substance S from the substance chamber **28** and a second, opposite surface **26b** configured for being heated by the heat transfer unit **24**.

Turning now to Figs. 2B and 2C, two additional examples of cartridge designs are shown, generally designate **20**' and **20**" respectively.

In the example shown in Fig. 2B, the sorption member **26**' is in the form of a ring having its first side rim **26**a' disposed within the substance chamber **28** so that it is immersed in the substance S. A second side rim **26**b' is disposed closer to the heat transfer unit **24** and configured for being heated thereby. It is observed that the sorption member **26**' does not occupy the entire space delimited between the substance chamber **28** and the heat transfer unit **24**, which constitutes, in this example, a vaporization chamber **23**.

Under this example, any substance S being vaporized owing to the heat provided by the heat transfer unit **24** first resides in the vaporization chamber **23** and is gradually and continuously removed therefrom via the channel **25** according to the user's puffs and sucking on the mouthpiece.

With attention being drawn to Fig. 2C, yet another example is shown, generally designated **20**" in which, instead of a ring-like sorption member **26**', the one used here is a sorption member **26**" which has extensions dipped in the substance S contained in the substance chamber **28**, gradually absorbing the substance S into the member **26**". The operation is similar to that previously described with respect to Fig. 2B.

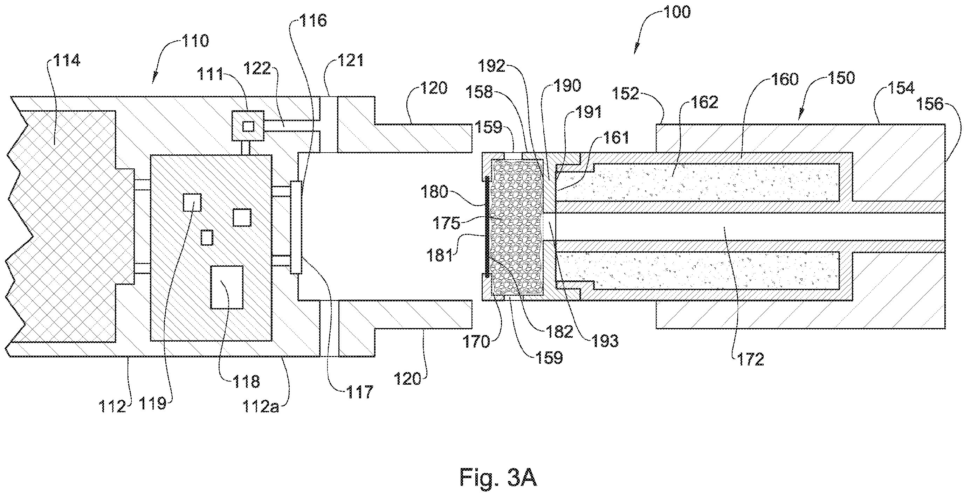

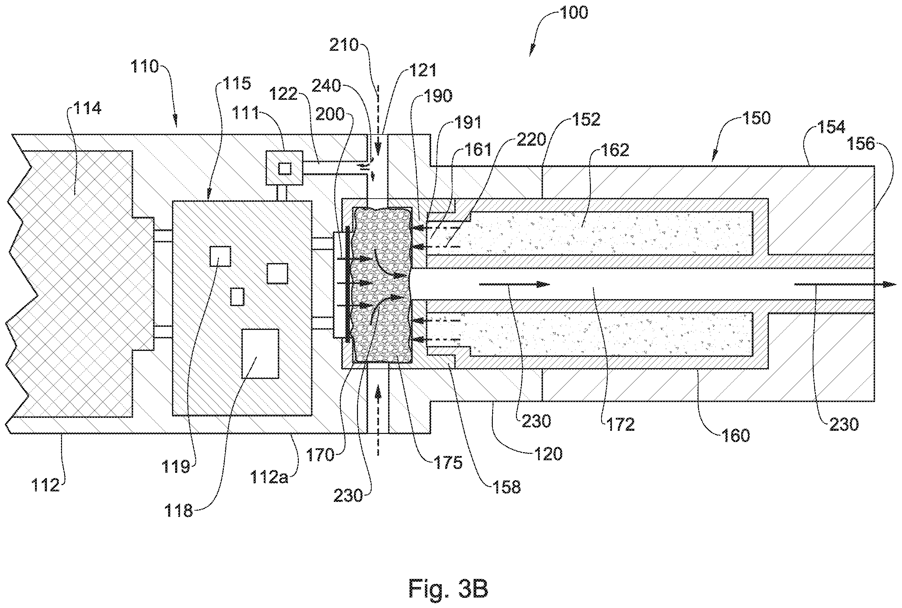

Reference is now made to Figs. 3A and 3B, illustrating a cross-sectional view of another example of an electronic cigarette, generally designated **100** and comprising a base part **110** and a cartridge **150**. In Fig. 3A the base part **110** and the cartridge **150** are assembled to each other, and being operated by a user.

The base part **110** comprises a cylindrical base housing **112** accommodating therein a battery **114** connected to a heating element **116** located at a first end **112a** of the base housing **112** and having an interface surface **117**. The battery **114** is configured for providing the heating element **116** with the necessary power for its operation, allowing it to become heated to a required temperature.

The cartridge **150** is structured of a cartridge housing **152** having a proximal end **154** with a mouthpiece **156** configured for being introduced into a user's mouth (not shown), and a distal end **158** configured to be introduced into the first end **112a** of the base housing **110.** The cartridge housing **152** includes a substance chamber **160** extending along its length and containing therein a substance **162** to be smoked. The substance chamber has a substance outlet **161** disposed at the distal end **158** of the cartridge housing **150**, and configured for allowing the substance **162** to escape from the substance chamber **160**.

The cartridge housing **152** further comprises a vaporization chamber 170 in communication with the substance outlet **161** for receiving the substance **162** therein, and a heat transfer unit **180** configured, when the cartridge is assembled to the base part **110**, for being heated by the heating element **116** of the base part **110** and for heating the substance **162** being provided via the substance outlet **161** for its vaporization in the vaporization chamber **170.** The vaporization chamber **170** is in fluid communication with a vaporization channel **172** extending from the vaporization chamber **170** to the mouthpiece **156.** The vaporization chamber **170** coextends with the substance chamber **160**, while the substance chamber **160** fully surrounds the vaporization channel **172**. The heat transfer unit **180** is perpendicular to the vaporization channel **172**. The heating element can include a temperature sensor (not shown) attached thereto or integrated therein for providing a signal determining the required temperature and heating rate of the heating element **116**, while the power for such heating is supplied by the battery **114**.

The heat transfer unit **180** has a first side **181** configured, when the cartridge **150** is assembled to the base part **110**, for interfacing a heating surface **117** of the heating element **116** for receiving heat therefrom, and an opposite second side **182** facing the vaporization chamber **170** for providing heat to the substrate being provided from the substrate outlet **161**.

The base housing **112** has an interfacing portion **120** disposed in proximity to the heating element **116** and configured for interfacing the distal end **158** of the cartridge housing **152** when the cartridge **150** is assembled to the base part **110**, so as to bring the heating element **116** to contact with the heat transfer unit **180.** In particular, in assembly of the cartridge **150** with the base part **110**, the heating surface **117** is fully matched with the first side **181** of the heat transfer unit **180** for transferring heat from the heating element **116** to the heat transfer unit **180**, and from the heat transfer unit **180** via the second side **182** to the vaporization chamber **170** in which the smoked substance is heated and vaporized. The configuration of the electronic cigarette is thus such that when the cartridge **150** is assembled to the base part **110**, the heating element **116** is separated from the vaporization chamber **170** by the heat transfer unit **180**.

The cartridge housing **154** further comprises a first air inlet **159** formed at the distal end **158** for establishing fluid communication between an exterior of the cartridge **150** and the vaporization chamber **170** for allowing passage of air into the vaporization chamber **170** for its vaporization therein with the substance **162** being supplied via the substance outlet **161**.

The interfacing portion **120** comprises a second air inlet **121** formed therein, and configured when the cartridge **150** is assembled to the base part **110** for establishing fluid communication between an exterior of the electronic cigarette **100** and the vaporization chamber **170** via the first air inlet **159** for allowing passage of air into the vaporization chamber **170** for its vaporization therein with the substance **162** being supplied via the substance outlet **161**. As shown in Fig. 3b, when the cartridge **150** is assembled to the base part **110**, said first air inlet **159** and said second air inlet **121** are inline with each other.

The vaporization chamber **170** includes a sorption member **175** disposed therein. The sorption member **175** is configured, on the one hand, for absorbing some of the substance **162**, and, on the other hand, for being heated by the heat transfer unit **180** thereby allowing the substance **162** absorbed therein to vaporize into the vaporization chamber **170** and escape from the vaporization chamber **170** via the vaporization channel **172** to the user via the mouthpiece **156**.

The cartridge housing **152** further comprises a porous member **190** interposed between the substance outlet **161** and the vaporization chamber **170**, so that the vaporization chamber **170** is interposed between the porous member **190** and the heat transfer unit **180**. The porous member **190** has a first surface **191** facing the substance outlet **161** and an opposite second surface **192** facing the sorption member **175** in the vaporization chamber **170,** for allowing passage of the substance **162** from said substance chamber **160** to the sorption member **175**. The second surface **192** is associated with the heat transfer unit **180** by facing its second side **182** and is separated from the heat transfer unit **180** by said vaporization chamber **170** for allowing introduction of the substance **160** from the second surface **192** into the sorption member **175** in the vaporization chamber **170** for its heating and thereby its vaporization therein. The porous member **190** is sized and shaped to fit the cross-section of the distal end **158** of the cartridge housing **154.**

The porous member **190** is formed with a hole **193** extending therethrough between the first surface **191** and the second surface **192** and establishing fluid communication between the vaporization chamber **170** and the mouthpiece **156** via the vaporization channel **172**, thereby allowing the vaporized substance to escape from the vaporization chamber **170**, and in particular, from the sorption member **175** via the mouthpiece **156** during smoking the vaporized substance by the user. During operation of the electronic cigarette **100**, the heat is configured to be delivered from the heat transfer unit **180** to the sorption member **175** by conduction.

It should be indicated that according to other examples, the sorption member **175** and the porous member **190** can be integrated in the same member, so that the sorption member is in direct contact with the substance outlet **161.**

Reference is now made specifically to Fig. 3B to explain the manner of operation of the electronic cigarette **100** during use by a user. In operation, the heating element **116** is heated up by the battery **114** and provides heat from its heating surface **117** to the first side **181** of the heat transfer unit **180** via **24** by conduction. The heat from the heat transfer unit is designated by an arrow **200.** During use of the electronic cigarette **100** by a user, the user inhales air which is provided to the vaporization chamber **170**, and in particular, to the sorption member **175** via the second air inlet **121** and the first air inlet **159**, and designated by an arrow **210**. Simultaneously, the smoked substance **160** is provided via the second surface **192** of the porous member **190** to the vaporization chamber **170** as well, and this substance is designated by an arrow **220.** The smoked substance **220** is mixed with the air **210** and heated by the heat **200** is the vaporization chamber **170**. The heating of the air **210** and the smoked substance **220** results in their vaporization in the sorption member **175** within the vaporization chamber **170**. The vaporized substance, which is designated by an arrow **230**, is afterwards transferred via the vaporization channel **172** to the mouthpiece **156**, and from there to the user.

During the above operation, the sorption member **175** is heated indirectly via the heat transfer unit **180** and not directly via the heating element **116**, leading to considerable advantages in terms of safety, temperature control and reliability of the electronic cigarette **100**.

The battery **114** is also connected to an electronic unit **115** including a processor **118** and a sensing unit **119**, for providing the required power for operation of the electronic unit **115** and its components. The processor **118** is connected to both the sensing unit **119** and the heating element **116**. The sensing unit **119** can be configured for identifying movement, orientation and spatial position of at least the base part **110**, and providing this information (hereinafter: spatial data) to the processor **118**. Based on the spatial data, the processor **118** can determine whether to turn the heating element **116** on/off, and/or if to set a certain operational regime of the electronic cigarette **100**, e.g. standby mode, regulation of vapor amount, timed smoking programs etc.

Thus, in operation, upon identification of a predetermined gesture of the user by the sensing unit **119**, this information is provided from the sensing unit **119** to the processor **118**, which then determines, based on said gesture, the proper operational mode of the electronic cigarette **100**. Upon determining such a mode, the processor **118**, which is directly linked to the heating element **116**, provides the latter with the proper operational signal determining the required temperature and heating rate of the heating element **116**, while the power for such heating is supplied by the battery **114**.

As a result, the electronic cigarette **100** can be configured for being operated using gestures and positions thereof, for example, a double-shake of the electronic cigarette **100** can indicate to the processor to turn the electronic cigarette **100** on/off where as holding the electronic cigarette **100** in a vertical orientation can indicate the processor to set a 'standby' mode for the electronic cigarette **100.** It should be appreciated that the sensing unit **119** can include various types of sensors (e.g. sound, motion, orientation, acceleration etc.), whereby the gestures required from the user in order to operate the electronic cigarette **100** can change correspondingly, e.g. (knocking the electronic cigarette against a surface, speed of motion eng.).

The base part **110** further comprises a puff detector **111** (e.g., a pressure sensor or a microphone), connected to the processor **118** for providing puff detection. The puff detector **111** is disposed in fluid communication with the second air inlet **121** via a third air inlet **122**, so that when air **210** passes along the second air inlet **210**, it also enters into the third air inlet **122**. The air that passes along the third air inlet **122** is shown in Fig. 3B by arrows **240**. During operation of the electronic cigarette, the puff detector **111** and the processor **118** are configured to identify air pressure changes of the air **240**, and performing different operations respectively. This operation include, for example, counting puffs for various purposes, turning off the cigarette when the puff number breathed is equivalent to a defined puff number and/or puffing time, and detecting the power with which the user breathes and thereby automatically adapting the quantity of the delivered smoked substance. The information provided by the puff detector **111** can allow the processor **118** to determine, based on the number of puffs and length of each puff, what is the estimated number of corresponding 'real life cigarettes' smoked by the user and alert him to the fact. For example, the processor **118** can be configured to provide a signal to the user that he has smoked over ten cigarettes and set an alert. Alternatively, the user may be able to set, in advance, a cigarette limit after which the electronic cigarette shuts down for a predetermined amount of time.

## Claims

1. An electronic cigarette (1; 100) comprising a base part (10; 110) and a cartridge (20; 20'; 150) configured to be assembled thereto,
said base part comprising a battery (14; 114) and at least one heating element (16; 116) connected to the battery, and
said cartridge comprising:
a substance chamber (28; 160) configured for containing therein a substance (S; 162) to be smoked, said substance chamber comprising a substance outlet (161);
a vaporization chamber (23; 170) in communication with the substance outlet for receiving said substance therein; and
at least one heat transfer unit (24; 180) configured, at least when the cartridge is assembled to the base part, for being heated by the heating element of the base part and for heating the substance being provided via said substance outlet for its vaporization in the vaporization chamber,
***characterized in that***
the cartridge further comprises a porous member (26; 26'; 26"; 190) interposed between the substance outlet and the vaporization chamber, and configured for allowing passage of said substance from said substance chamber to said vaporization chamber.

2. An electronic cigarette according to Claim 1, wherein the cartridge further comprises a sorption member (26; 26'; 26"; 175) at least partially disposed within the vaporization chamber.

3. An electronic cigarette according to Claim 1 or 2, wherein the heat transfer unit has a first side (181) configured, when the cartridge is assembled to the base part, for interfacing the heating element for receiving heat therefrom, and a second side (182) facing the substance outlet for providing heat to the substance being provided from the substance outlet.

4. An electronic cigarette according to Claim 1, 2 or 3, wherein, when the cartridge is assembled to the base part, the heating element is separated from the vaporization chamber by the heat transfer unit.

5. An electronic cigarette according to any one of Claims 1 to 4, wherein said vaporization chamber is interposed between the porous member and the heat transfer unit.

6. An electronic cigarette according to any one of Claims 1 to 5, wherein the porous member has a first surface (191) facing the substance outlet, and a second surface (192) facing the heat transfer unit.

7. An electronic cigarette according to Claim 6, wherein the porous member is formed with a hole (193) extending therethrough between the first surface and the second surface and establishing fluid communication between the vaporization chamber and a mouthpiece (156) of the cartridge via a vaporization channel (172), thereby allowing the vaporized substance to escape from the vaporization chamber via the mouthpiece.

8. An electronic cigarette according to any one of Claims 1 to 7, wherein the porous member is sized and shaped to fit the cross-section of one of the vaporization chamber and substance chamber.

9. An electronic cigarette according to any one of Claims 1 to 8, wherein the cartridge further comprises: a cartridge housing (22; 152) at least partially including said substance chamber and said vaporization chamber, and a vaporization channel (25; 172) extending along the cartridge housing and in fluid communication with the vaporization chamber; and wherein the cartridge housing has a proximal end (22b; 154) configured with a mouthpiece (30; 156) which is in fluid communication with the vaporization chamber via the vaporization channel and a distal end (22a; 158) associated with the heat transfer unit.

10. An electronic cigarette according to Claim 9, wherein said heat transfer unit is disposed at said distal end.

11. An electronic cigarette according to Claim 9 or 10, wherein said heat transfer unit is perpendicular to the vaporization channel.

12. An electronic cigarette according to any one of Claims 9 to 11, wherein said cartridge housing comprises a first air inlet (159) formed therein for establishing fluid communication between an exterior of the cartridge and the vaporization chamber for allowing passage of air into the vaporization chamber for its vaporization therein with the substance.

13. An electronic cigarette according to Claim 12, wherein said first air inlet is disposed in proximity to the distal end of the cartridge.

14. An electronic cigarette according to any one of Claims 9 to 13, wherein the base part (110) has a base housing (112) having an interfacing portion (120) associated with the heating element and configured for interfacing the distal end of the cartridge housing when the cartridge is assembled to the base part, so as to bring the heating element into proximity or contact with the heat transfer unit.

15. An electronic cigarette according to Claim 14, when dependent on Claim 12, wherein said interfacing portion comprises a second air inlet (121) formed therein, and configured at least when the cartridge is assembled to the base part for establishing fluid communication between an exterior of the electronic cigarette and the vaporization chamber via the first air inlet for allowing passage of air into the vaporization chamber for its vaporization therein with the substance.

16. An electronic cigarette according to Claim 15, wherein when the cartridge is assembled to the base part, said first air inlet and said second air inlet are inline with each other.

17. An electronic cigarette according to any one of Claims 1 to 16, wherein the battery is configured for electrically heating the heating element of the base part, to provide its heat by conduction to the heat transfer unit.

18. An electronic cigarette according to any one of Claims 1 to 17, wherein the base part further comprises a processor (18; 118) associated with electrical components of the electronic cigarette, including said battery.

19. An electronic cigarette according to Claim 18, wherein the base part further comprises a sensing unit (15; 119) connected to the processor for identifying movement or positioning of the electronic cigarette and thereby allow a gesture-based operation of the electronic cigarette.

20. An electronic cigarette according to Claim 18 or 19, wherein the base part comprises a pressure sensor or a microphone, connected to the processor for providing puff detection.

21. An electronic cigarette according to Claim 20, wherein the processor is configured for turning off the cigarette when the puff number breathed in is equivalent to a defined puff number and/or puffing time.

## Patentansprüche

1. Elektronische Zigarette (1; 100) mit einem Unterteil (10; 110) und einer Kartusche (20; 20'; 150), welche so konfiguriert ist, dass sie daran montiert werden kann,
wobei das Unterteil eine Batterie (14; 114) und wenigstens ein Heizelement (16; 116), welches mit der Batterie verbunden ist, aufweist und
wobei die Kartusche folgende Merkmale aufweist:
eine Substanzkammer (28; 160), welche ausgebildet ist, um darin eine Substanz (S; 162), welche geraucht werden soll zu enthalten, wobei die Substanzkammer einen Substanzauslass (161) aufweist;
eine Verdampfungskammer (23; 170) in Verbindung mit dem Substanzauslass zum Aufnehmen der Substanz hierin und
wenigstens eine Wärmeübertragungseinheit (24; 180), welche ausgebildet ist, wenigstens wenn die Kartusche an dem Unterteil montiert ist, durch das Heizelement des Unterteils aufgeheizt zu werden und zum Heizen der Substanz, welche über den Substanzauslass für ihre Verdampfung in dem Verdampferbauteil vorgesehen ist,
**dadurch gekennzeichnet, dass**
die Kartusche außerdem ein poröses Bauteil (26; 26'; 26"; 190) aufweist, welches zwischen dem Substanzauslass und der Verdampfungskammer angeordnet ist und so konfiguriert ist, dass es einen Durchfluss der genannten Substanz von der genannten Substanzkammer zu der genannten Verdampfungskammer ermöglicht.

2. Elektronische Zigarette nach Anspruch 1, bei der die Kartusche ein Sorptionselement (26; 26'; 26"; 175) aufweist, welches wenigstens teilweise in der Verdampfungskammer angeordnet ist.

3. Elektronische Zigarette nach Anspruch 1 oder 2, bei der die Wärmeübertragungseinheit eine erste Seite (181) hat, die wenn die Kartusche an dem Unterteil montiert ist, zum Ankoppeln des Heizelements zur Aufnahme von Wärme hiervon ausgebildet ist, und eine zweite Seite (182) hat, welche zu dem Substanzauslass zeigt, um Wärme zu der Substanz zu liefern, welche von dem Substanzauslass geliefert wird.

4. Elektronische Zigarette nach Anspruch 1, 2 oder 3, bei der die Kartusche an dem Unterteil montiert ist, wobei das Heizelement von der Verdampfungskammer durch die Wärmeübertragungseinheit getrennt ist.

5. Elektronische Zigarette nach einem der Ansprüche 1 bis 4, bei der die Verdampfungskammer zwischen dem porösen Element und der Wärmeübertragungseinheit angeordnet ist.

6. Elektronische Zigarette nach einem der Ansprüche 1 bis 5, bei der das poröse Element eine erste Oberfläche (191) hat, welche zu dem Substanzauslass zeigt, und eine zweite Oberfläche (192) aufweist, welche zu der Wärmeübertragungseinheit zeigt.

7. Elektronische Zigarette nach Anspruch 6, bei der das poröse Element mit einem Loch (193) gebildet ist, welches sich dort hindurch erstreckt zwischen der ersten Oberfläche und der zweiten Oberfläche und eine Fluidkommunikation zwischen der Verdampfungskammer und einem Mundstück (156) der Kartusche über einem Verdampfungskanal (172) etabliert, wodurch ermöglicht wird, dass verdampfte Substanz aus der Verdampfungskammer über das Mundstück entweichen kann.

8. Elektronische Zigarette nach einem der Ansprüche 1 bis 7, bei der das poröse Element bemaßt und geformt ist, um auf den Querschnitt der Verdampfungskammer oder der Substanzkammer zu passen.

9. Elektronische Zigarette nach einem der Ansprüche 1 bis 8, bei der die Kartusche weiter folgende Merkmale aufweist: ein Kartuschengehäuse (22; 152), welches wenigstens teilweise die Substanzkammer und die Verdampfungskammer einschließt, und einen Verdampfungskanal (25; 172), welcher sich entlang des Kartuschengehäuses erstreckt und in Fluidkommunikation mit der Verdampfungskammer steht, und wobei das Kartuschengehäuse ein proximales Ende (22b; 154) hat, welches mit einem Mundstück (30; 156) geformt ist, welches mit der Verdampfungskammer über den Verdampfungskanal und ein distales Ende (22a; 158) in Fluidkommunikation steht, welches mit der Wärmeübertragungseinheit verbunden ist hat.

10. Elektronische Zigarette nach Anspruch 9, bei der die Wärmeübertragungseinheit an dem distalen Ende angeordnet ist.

11. Elektronische Zigarette nach Anspruch 9 oder 10, bei der die Wärmeübertragungseinheit senkrecht zu dem Verdampfungskanal steht.

12. Elektronische Zigarette nach einem der Ansprüche 9 bis 11, bei der das Kartuschengehäuse einen ersten Lufteinlass (159) aufweist, welcher dort gebildet ist, um eine Fluidkommunikation zwischen einem äußeren der Kartusche und der Verdampfungskammer zu etablieren, um den Durchfluss von Luft in die Verdampfungskammer zu ermöglichen, um diese darin mit der Substanz zu verdampfen.

13. Elektronische Zigarette nach Anspruch 12, bei der der erste Lufteinlass in der Nähe zu dem distalen Ende der Kartusche angeordnet ist.

14. Elektronische Zigarette nach einem der Ansprüche 9 bis 13, bei der das Unterteil (110) ein Grundgehäuse (112) hat, welches einen Koppelabschnitt (120) aufweist, welcher mit dem Heizelement verbunden ist und zum Ankoppeln des distalen Endes des Kartuschengehäuses konfiguriert ist, wenn die Kartusche an dem Unterteil montiert ist, um das Heizelement in die Nähe oder in Kontakt mit der Wärmeübertragungseinheit zu bringen.

15. Elektronische Zigarette nach Anspruch 14, soweit dieser von Anspruch 12 abhängt, bei welcher der Koppelabschnitt einen zweiten darin geformten Lufteinlass (121) aufweist und so ausgebildet ist, dass er wenigstens, wenn die Kartusche an dem Unterteil montiert ist, eine Fluidkommunikation zwischen einem äußeren der elektronischen Zigarette und der Verdampfungskammer über den ersten Lufteinlass zu etablieren, um einen Durchfluss von Luft in die Verdampfungskammer für deren Verdampfung darin mit der Substanz zu ermöglichen.

16. Elektronische Zigarette nach Anspruch 15, bei der, wenn die Kartusche an dem Unterteil montiert ist, der erste Lufteinlass und der zweite Lufteinlass zueinander in Reihe liegen.

17. Elektronische Zigarette nach einem der Ansprüche 1 bis 16, bei der die Batterie zum elektrischen Heizen des Heizelements des Unterteils konfiguriert ist, um ihre Wärme durch Konduktion zu der Wärmeübertragungseinheit zu liefern.

18. Elektronische Zigarette nach einem der Ansprüche 1 bis 17, bei der das Unterteil außerdem einen Prozessor (18; 118) aufweist, welcher mit den elektrischen Komponenten der elektronischen Zigarette einschließlich der Batterie verbunden ist.

19. Elektronische Zigarette nach Anspruch 18, bei der das Unterteil außerdem eine Messfühlereinheit (15; 119) aufweist, welche mit dem Prozessor verbunden ist, um eine Bewegung oder Positionierung der elektronischen Zigarette zu identifizieren und hierdurch einen Betrieb der elektronischen Zigarette auf der Basis einer Handbewegung zu ermöglichen.

20. Elektronische Zigarette nach Anspruch 18 oder 19, bei der das Unterteil einen Drucksensor oder ein Mikrofon aufweist, welche mit dem Prozessor verbunden sind, um eine Zugerkennung zu schaffen.

21. Elektronische Zigarette nach Anspruch 20, bei der Prozessor konfiguriert ist, um die Zigarette abzuschalten, wenn die eingeatmete Zuganzahl äquivalent ist zu einer definierten Zuganzahl und/oder Zugzeit.

## Revendications

1. Cigarette électronique (1 ; 100) comprenant une partie de base (10 ; 110) et une cartouche (20 ; 20' ; 150) configurée pour être assemblée dessus, ladite partie de base comprenant une batterie (14 ; 114) et au moins un élément chauffant (16 ; 116) relié à la batterie, et
ladite cartouche comprenant :
une chambre de substance (28 ; 160) configurée pour contenir une substance (S ; 162) à fumer, ladite chambre de substance comprenant une évacuation de substance (161) ;
une chambre de vaporisation (23 ; 170) en communication avec l'évacuation de substance pour recevoir en son sein ladite substance ; et
au moins une unité de transfert de chaleur (24 ; 180) configurée, au moins lorsque la cartouche est assemblée sur la partie de base, pour être chauffée par l'élément chauffant de la partie de base et pour chauffer la substance fournie via ladite évacuation de substance en vue de sa vaporisation dans la chambre de vaporisation,
**caractérisée en ce que**
la cartouche comprend en outre un élément poreux (26 ; 26' ; 26" ; 190) interposé entre l'évacuation de substance et la chambre de vaporisation, et configuré pour permettre le passage de ladite substance entre ladite chambre de substance et ladite chambre de vaporisation.

2. Cigarette électronique selon la revendication 1, dans laquelle la cartouche comprend en outre un élément de sorption (26 ; 26' ; 26" ; 175) au moins partiellement disposé dans la chambre de vaporisation.

3. Cigarette électronique selon la revendication 1 ou 2, dans laquelle l'unité de transfert de chaleur présente un premier côté (181) configuré, lorsque la cartouche est assemblée sur la partie de base, pour faire interface avec l'élément chauffant afin de recevoir de la chaleur de la part de celui-ci, et un second côté (182) tourné vers l'évacuation de substance afin de fournir de la chaleur à la substance fournie par l'évacuation de substance.

4. Cigarette électronique selon la revendication 1, 2 ou 3, dans laquelle, lorsque la cartouche est assemblée sur la partie de base, l'élément chauffant est séparé de la chambre de vaporisation par l'unité de transfert de chaleur.

5. Cigarette électronique selon l'une quelconque des revendications 1 à 4, dans laquelle ladite chambre de vaporisation est interposée entre l'élément poreux et l'unité de transfert de chaleur.

6. Cigarette électronique selon l'une quelconque des revendications 1 à 5, dans laquelle l'élément poreux présente une première surface (191) tournée vers l'évacuation de substance, et une seconde surface (192) tournée vers l'unité de transfert de chaleur.

7. Cigarette électronique selon la revendication 6, dans laquelle l'élément poreux est formé avec un orifice (193) qui s'étend entre la première surface et la seconde surface et qui crée une communication de fluide entre la chambre de vaporisation et un embout (156) de la cartouche via un canal de vaporisation (172), afin de permettre à la substance vaporisée de s'échapper de la chambre de vaporisation via l'embout.

8. Cigarette électronique selon l'une quelconque des revendications 1 à 7, dans laquelle l'élément poreux est dimensionné et formé pour s'adapter à la section transversale de l'une de la chambre de vaporisation et de la chambre de substance.

9. Cigarette électronique selon l'une quelconque des revendications 1 à 8, dans laquelle la cartouche comprend en outre : un logement de cartouche (22 ; 152) qui comprend au moins partiellement ladite chambre de substance et ladite chambre de vaporisation, et un canal de vaporisation (25 ; 172) qui s'étend le long du logement de cartouche et en communication de fluide avec la chambre de vaporisation ; et dans laquelle le logement de cartouche possède une extrémité proximale (22b ; 154) configurée avec un embout (30 ; 156) qui est en communication de fluide avec la chambre de vaporisation via le canal de vaporisation, et une extrémité distale (22a ; 158) associée à l'unité de transfert de chaleur.

10. Cigarette électronique selon la revendication 9, dans laquelle ladite unité de transfert de chaleur est disposée au niveau de ladite extrémité distale.

11. Cigarette électronique selon la revendication 9 ou 10, dans laquelle ladite unité de transfert de chaleur est perpendiculaire au canal de vaporisation.

12. Cigarette électronique selon l'une quelconque des revendications 9 à 11, dans laquelle ledit logement de cartouche comprend une première admission d'air (159) formée à l'intérieur afin de créer une communication de fluide entre un extérieur de la cartouche et la chambre de vaporisation pour permettre le passage de l'air dans la chambre de vaporisation en vue de sa vaporisation à l'intérieur avec la substance.

13. Cigarette électronique selon la revendication 12, dans laquelle ladite première admission d'air est disposée à proximité de l'extrémité distale de la cartouche.

14. Cigarette électronique selon l'une quelconque des revendications 9 à 13, dans laquelle la partie de base (110) possède un logement de base (112) ayant une partie d'interface (120) associée à l'élément chauffant et configuré pour faire interface avec l'extrémité distale du logement de cartouche lorsque la cartouche est assemblée sur la partie de base, de façon à amener l'élément chauffant à proximité de ou en contact avec l'unité de transfert de chaleur.

15. Cigarette électronique selon la revendication 14, lorsqu'elle dépend de la revendication 12, dans laquelle ladite partie d'interface comprend une seconde admission d'air (121) formée à l'intérieur, et configurée, au moins lorsque la cartouche est assemblée sur la partie de base, pour créer une communication de fluide entre un extérieur de la cigarette électronique et la chambre de vaporisation via la première admission d'air afin de permettre le passage de l'air dans la chambre de vaporisation en vue de sa vaporisation à l'intérieur avec la substance.

16. Cigarette électronique selon la revendication 15, dans laquelle, lorsque la cartouche est assemblée sur la partie de base, ladite première admission d'air et ladite seconde admission d'air sont alignées l'une avec l'autre.

17. Cigarette électronique selon l'une quelconque des revendications 1 à 16, dans laquelle la batterie est configurée pour chauffer électriquement l'élément chauffant de la partie de base, afin de fournir sa chaleur par conduction à l'unité de transfert de chaleur.

18. Cigarette électronique selon l'une quelconque des revendications 1 à 17, dans laquelle la partie de base comprend en outre un processeur (18 ; 118) associé aux composants électriques de la cigarette électronique, y compris ladite batterie.

19. Cigarette électronique selon la revendication 18, dans laquelle la partie de base comprend en outre une unité de détection (15 ; 119) reliée au processeur afin d'identifier le mouvement ou le positionnement de la cigarette électronique et de permettre un fonctionnement de la cigarette électronique sur la base d'un geste.

20. Cigarette électronique selon la revendication 18 ou 19, dans laquelle la partie de base comprend un capteur de pression ou un microphone, relié au processeur afin d'assurer une détection de bouffée.

21. Cigarette électronique selon la revendication 20, dans laquelle le processeur est configuré pour éteindre la cigarette lorsque le nombre de bouffées aspirées est équivalent à un nombre de bouffées et/ou une durée de bouffée défini(e).
